(19) Europäisches Patentamt
European Patent Office
Office européen des brevets

(11) Veröffentlichungsnummer: **0 124 911 B1**

# (12) EUROPÄISCHE PATENTSCHRIFT

(45) Veröffentlichungstag der Patentschrift: **31.07.91**

(51) Int. Cl.5: **C07D 277/34**, A61K 31/425

(21) Anmeldenummer: **84105322.6**

(22) Anmeldetag: **10.05.84**

(54) **4-Oxo-thiazolidin-2-yliden-acetamid-Derivate, Verfahren zu deren Herstellung und diese enthaltende Arzneimittel zur Bekämpfung von Erkrankungen des Zentralnervensystems.**

(30) Priorität: **10.05.83 DE 3317000**

(43) Veröffentlichungstag der Anmeldung:
**14.11.84 Patentblatt 84/46**

(45) Bekanntmachung des Hinweises auf die Patenterteilung:
**31.07.91 Patentblatt 91/31**

(84) Benannte Vertragsstaaten:
**AT BE CH DE FR GB IT LI LU NL SE**

(56) Entgegenhaltungen:
**FR-M- 2 726**

**Dictionnaire de la chimie, 30 edition, Seite 60, IUPAC 1979 Rule C-812-C-813**

(73) Patentinhaber: **GÖDECKE AKTIENGESELLSCHAFT**
**Salzufer 16**
**W-1000 Berlin 10(DE)**

(72) Erfinder: **Satzinger, Gerhard, Dr.**
**Im Mattenbühl 7**
**W-7809 Denzlingen(DE)**
Erfinder: **Herrmann, Manfred, Dr.**
**Wolfweg 25**
**W-7811 St. Peter(DE)**
Erfinder: **Fritschi, Edgar, Dr.**
**Am Scheuerwald 2**
**W-7811 St. Peter(DE)**
Erfinder: **Hartenstein, Johannes, Dr.**
**Fohrenbühl 23**
**W-7801 Stegen-Wittental(DE)**
Erfinder: **Bartoszyk, Gerd**
**Buchenweg 6**
**W-7808 Waldkirch(DE)**

(74) Vertreter: **Henkel, Feiler, Hänzel & Partner**
**Möhlstrasse 37**
**W-8000 München 80(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft neue 4-Oxo-thiazolidin-2-yliden-acetamid-derivate der allgemeinen Formel I

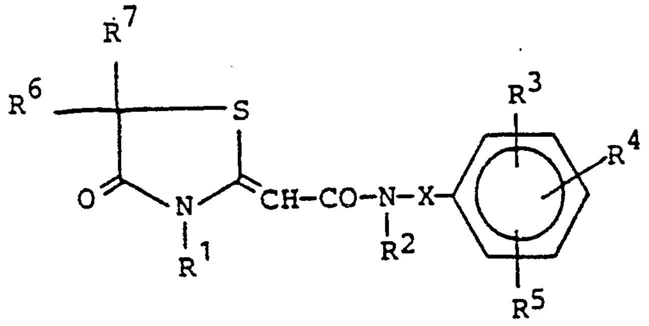

(I),

in welcher

$R^1$ ein Wasserstoffatom, einen gesättigten oder ungesättigten Alkylrest mit 1 bis 3 Kohlenstoffatomen, einen Phenyl- oder Benzylrest, $R^2$ ein Wasserstoffatom, eine Methyl- oder Ethylgruppe oder einen Dialkylaminoalkylrest mit insgesamt bis zu 7 Kohlenstoffatomen, X einen Bindestrich oder eine geradkettige oder verzweigte Alkylenkette mit 1 - 3 Kohlenstoffatomen und $R^3$, $R^4$ und $R^5$ die gleich oder verschieden sein können, ein Wasserstoffatom, eine Methyl-, Ethyl-, Methoxy-, Ethoxy-, Methylthio- oder Ethylthiogruppe, wobei zwei benachbarte Reste gemeinsam auch eine Methylendioxygruppe bilden können, ein Fluor-, Chlor-, Brom-, Jodatom, eine Hydroxygruppe oder eine Nitrogruppe oder eine Carboxylgruppe oder einen Niederalkoxycarbonylrest mit bis zu 5 Kohlenstoffatomen und $R^6$ und $R^7$ Wasserstoff, eine Methyl- oder eine Ethylgruppe bedeutet.

Ganz besonders bevorzugt sind Verbindungen der allgemeinen Formel I, in welcher $R^1$ ein Wasserstoffatom, einen Methyl-, Ethyl-, Allyl- oder Benzylrest, $R^2$ ein Wasserstoffatom, einen Methylrest, X einen Bindestrich oder eine Ethylidengruppe und $R^3$, $R^4$ und $R^5$, die gleich oder verschieden sein können, ein Wasserstoffatom, einen Methylrest oder ein Chlor- oder Bromatom und $R^6$ und $R^7$ ein Wasserstoffatom bedeutet.

En weiterer Gegenstand der Erfindung ist ein Verfahren zur Herstellung von Verbindungen der allgemeinen Formel I , dadurch gekennzeichnet, daß man entweder

a) eine Verbindung der allgemeinen Formel II

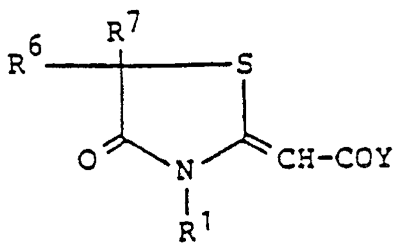

(II),

in welcher $R^1$, $R^6$ und $R^7$ die obengenannte Bedeutung haben und y eine reaktive Gruppe darstellt in an sich bekannter Weise mit einer Verbindung der allgemeinen Formel (III)

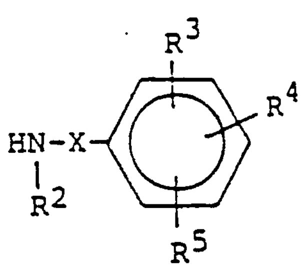

(III),

in welcher die Reste $R^2$, $R^3$, $R^4$ und $R^5$ und X die obengenannte Bedeutung haben,

umsetzt und gegebenenfalls anschließend N-alkyliert oder
b) eine Verbindung der allgemeinen Formel IV

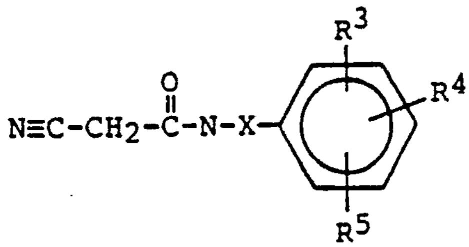

(IV),

in welcher die Reste $R^3$, $R^4$ und $R^5$ sowie X die obengenannte Bedeutung haben, mit einem Thioglykolsäureester der allgemeinen Formel V

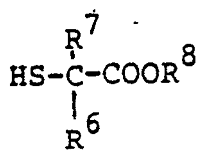

(V),

in welcher $R^6$ und $R^7$ die obengenannte Bedeutung haben und $R^8$ einen niederen Alkylrest mit bis zu 5 Kohlenstoffatomen darstellt,
umsetzt, die erhaltenen Verbindungen der allgemeinen Formel VI

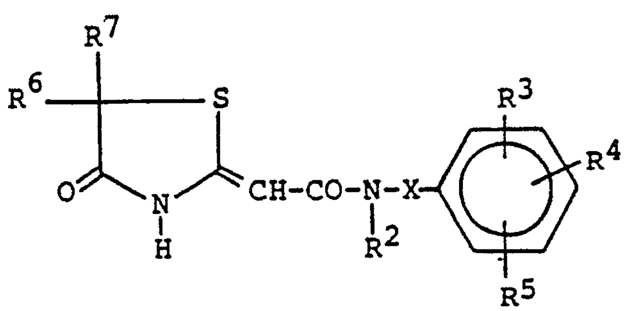

(VI),

in welcher $R^2$, $R^3$, $R^4$, $R^5$, $R^6$ und $R^7$ sowie X die obengenannte Bedeutung haben, gewünschtenfalls in an sich bekannter Weise anschließend N-alkyliert und die so erhaltenen Verbindungen der allgemeinen Formel I in an sich bekannter Weise isoliert.

Die Verbindungen der allgemeinen Formel I sind wertvolle Arzneimittel mit ungewöhnlich breitem antikonvulsivem Wirkungsprofil und hervorragender Verträglichkeit. Sie sind insbesondere für eine umfassende, antiepileptische Therapie geeignet.

Der Begriff der Epilepsie ist eine kollektive Bezeichnung für eine Klasse chronischer Erkrankungen des zentralen Nervensystems, welche das Auftreten episodischer Anfälle mit abnormalen motorischen ("Krämpfe"), sensorischen und psychischen Phänomenen gemeinsam haben.

Die Häufigkeit der Epilepsien ist überraschend groß und liegt bei ca. 0,5 % der Bevölkerung. Der Formenreichtum der Epilepsien zwingt zu einer Pharmakotherapie, die durch die Notwendigkeit einer oft wechselnden, differenzierten Behandlung mit unterschiedlichen Pharmaka sehr kompliziert wird.

Antiepileptische Substanzen gehören zu verschiedenartigen chemischen Klassen. Diese umfassen Barbiturate, Pyrimidone, Hydantoine, Oxazolidindione, Succinimide, Benzodiazepine, Iminostilbene und Valproate. Entsprechend ihren Wirkungen in Labormodellen sind Vertreter dieser Klassen mehr oder weniger zur Therapie partieller (fokaler), generalisierter, temporaler oder myoklonaler Anfälle und Absencen geeignet. Ein ideales Antiepileptikum wäre demnach eine Substanz, die alle Arten von Anfällen und Absencen zu unterdrücken in der Lage ist und keine unerwünschten Nebeneffekte erzeugt.

Ein Nachteil der bekannten Antiepileptika ist, daß sie beim Versuch, Anfälle zu kontrollieren, oft versagen und darüber hinaus ZNS-Funktionsstörungen und andere Nebenwirkungen bis hin zur aplastischen Anämie verursachen können (Goodman, Gilman, The Pharmacological Basis of Therapeutics - 6. Ed.

1980, S. 451 ff). Selbst wenn man bekannte Antikonvulsiva in geeigneter Auswahl und Kombination anwendet, können bei nur 50 % der behandelten Patienten auftretende Anfälle unter Kontrolle gebracht werden. Selbst im Erfolgsfalle müssen jedoch in der Regel unangenehme Nebeneffekte in Kauf genommen werden.

Die Verbindungen der allgemeinen Formel I zeichnen sich nun nicht nur durch eine außerordentliche Breite ihres Wirkungsspektrums aus, sondern auch durch eine hohe Potenz und durch eine extrem gute Verträglichkeit. Obwohl diese Befunde erst durch Tierversuche gestützt werden können, ist zu erwarten, daß die Verbindungen der Erfindung einen erheblichen Fortschritt bei der Behandlung der Epilepsie in ihren vielfältigen Erscheinungsformen bedeuten.

Die Verbindungen der allgemeinen Formel I können aufgrund der C-C-Doppelbindung am Thiazolidinring in einer Z- und E-Konfiguration vorkommen. Die vorliegende Anmeldung schließt beide Formen ein.

Die Ausgangsverbindungen II und III sind bekannt oder können nach bekannten Methoden hergestellt werden. Verbindungen der allgemeinen Formel II, in welcher $R^1$ ein Wasserstoffatom ist, können, ausgehend von den bekannten Säuren ($R^1$ = H, Y = OH) durch Alkylierung am Ringstickstoff nach vorhergehender Veresterung mittels niederer aliphatischer Alkohole und anschließender vorsichtige Hydrolyse erhalten werden (Annalen 665 (1963) S. 150 ff).
In besonders vorteilhafter Weise werden dabei die entsprechenden t-Butylester (Y = tert. Butoxy-Gruppe) eingesetzt.

Das Verfahren a) wird bevorzugt in einem aprotisch bipolaren Lösungsmittel, wie z. B. Dichlormethan, Chloroform oder Dimethylformamid oder in Gemischen solcher Lösungsmittel durchgeführt.

Als reaktive Gruppen kommen z. B. Anhydride, Alkylester, gemischte Anhydride, insbesondere jedoch die Halogenide in Frage.
Die Reaktion läßt sich jedoch auch unter Einsatz von Alkylestern durchführen (Y = niedere Alkoxy-Gruppe).

Die besonders reaktionsfähigen Säurechloride werden hergestellt, indem man die freie Säure (Y = OH) mit einem geeigneten Säurehalogenid, wie z. B. Thionylchlorid, Oxalylchlorid oder Phosphorpentachlorid umsetzt.

Die Verbindungen der allgemeinen Formel IV lassen sich - ausgehend von Cyanessigsäureestern - wie z. B. Cyanessigsäureethylester mit entsprechenden Anilinderivaten der allgemeinen Formel VII

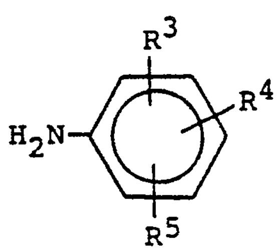

(VII),

in welcher $R^3$ $R^4$ und $R^5$ die obengenannte Bedeutung haben nach allgemein bekannten Verfahren, z. B. durch Erhitzen der Reaktionskomponenten unter Abdestillation des freiwerdenden Esteralkohols herstellen.

Die Verbindungen der allgemeinen Formel I, in welchen $R^1$ ein Wasserstoffatom bedeutet, werden vorzugsweise nach Verfahren b) hergestellt, da das Verfahren a) in Einzelfällen unbefriedigende Ausbeuten ergibt.

Die erfindungsgemäßen Verbindungen I können in flüssiger oder fester Form enteral oder parenteral appliziert werden. Als Injektionsmedium kommen vor allem wäßrige Phasen zur Anwendung, welche die üblichen zusätze wie Stabilisierungsmittel und Lösungsvermittler enthalten. Feste Trägerstoffe sind z. B. Stärke, Lactose, Mannit, Methylcellulose, Talkum, hochdisperse Kieselsäuren, höhermolekulare Fettsäuren (wie Stearinsäure), Gelatine, Agar-Agar, Kalziumphosphat, Magnesiumstearat, tierische und pflanzliche Fette, feste hochmolekulare Polymere (wie Polyäthylenglykole); für orale Applikation geeignete Zubereitungen können gewünschtenfalls Geschmacks- und/oder Süßstoffe enthalten.
Die Dosierung der erfindungsgemäßen Verbindungen richtet sich nach Art und Schwere der jeweiligen Erkrankung. Die orale Einzeldosis beträgt etwa 10 - 200 mg.

Die folgenden Beispiele dienen der näheren Erläuterung der Erfindung:

**Beispiel 1**

(Z)-(4-Oxo-thiazolidin-2-yliden)-N-(2,6-dimethylphenyl)-acetamid

4,2 g (35 mMol) Thioglycolsäure-ethylester werden mit 6,6 g (35 mMol) Cyanessigsäure-2,6-dimethylanilid und 0,13 g (1 mMol) Kaliumcarbonat vermischt.

Anschließend werden 15 ml Ethanol zugegeben und das Gemisch unter Rühren 3,5 Stunden zum Sieden erhitzt. Man gibt dann weitere 15 ml Ethanol zu und kühlt auf 40 °C ab. Der ausgefallene Niederschlag wird abgesaugt, das Rohprodukt aus So %iger wäßriger Essigsäure umkristallisiert, mit Wasser neutral gewaschen und im Vakuum getrocknet. Man erhält (Z)-(4-Oxo-thiazolidin-2-yliden)-N-(2,6-dimethylphenyl)-acetamid; Schmp. 250 °C (Zers.)

Das als Ausgangsprodukt verwendete Cyanessigsäure-2,6-dimethylanilid wird wie folgt hergestellt:

11,3 g (0,1 Mol) Cyanessigsäure-ethylester und 15,7 g (0,13 Mol) 2,6-Dimethylanilin werden vermischt und unter Rühren auf 170 °C erhitzt. Das entstehende Ethanol wird dabei laufend abdestilliert; hierbei steigt die Temperatur bis auf 195 °C an. Nach 6 Stunden ist die Reaktion beendet.

Der Kolbeninhalt wird nach dem Erkalten aus 350 ml Ethanol umkristallisiert. Man erhält das Cyanessigsäure 2,6-dimethylanilid in Form farbloser Kristalle vom Schmp. 204 - 206 °C.

## Beispiel 2

(Z)-(3-Methyl-4-oxo-thiazolidin-2-yliden)-N-(2,6-dimethylphenyl)acetamid

### Variante A

0,1 Mol (z)-(3-Methyl-4-oxo-thiazolidin-2-yliden)essigsäure werden in einer Mischung von 5oo ml Dichlormethan und 5 ml Dimethylformamid gelöst und 10 ml Thionylchlorid, gelöst in 30 ml Dichlormethan, innerhalb von 45 Minuten bei Raumtemperatur zugetropft. Man läßt das Reaktionsgemisch noch 15 Minuten bei dieser Temperatur rühren und dampft die nunmehr braune Lösung im Vakuum ein. Der Rückstand wird in 500 ml Dichlormethan gelöst. In die filtrierte Lösung tropft man dann innerhalb von 30 Minuten eine Lösung von 0,2 Mol 2,6-Dimethylanilin in 3o ml Dichlormethan bei Raumtemperatur zu und rührt weitere 4 Stunden bei 40 °C.

Der ausgefallene Niederschlag wird abgesaugt und aus 50 %iger wäßriger Essigsäure umkristallisiert, mit Wasser neutral gewaschen und im Vakuum getrocknet.

Man erhält (Z)-(3-Methyl-4-oxo-thiazolidin-2-yliden)-N-(2,6-dimethylphenyl) acetamid. Schmp. 250,4 °C (Zers.)

Die als Ausgangsprodukt verwendete (Z)-(3-Methyl-4-oxothiazolidin-2-yliden)essigsäure wird wie folgt hergestellt:

20 g (Z)-(3-Methyl-4-oxo-thiazolidin-2-yliden)essigsäure-t-butylester (Annalen 665 (1963) S. 150 ff) werden in 45 ml Eisessig gelöst und bei 0 °C mit 8 ml einer 33 %igen Lösung von Bromwasserstoff in Eisessig versetzt. Nach 15 Minuten wird Eiswasser zugesetzt und die ausgefallene Säure abgesaugt. Da sich das Endprodukt rasch zersetzt, wird schnell aus Isopropanol umkristallisiert. Die erhaltene freie Säure kann unmittelbar weiterverwendet werden.

### Variante B

0,020 Mol (Z)-(4-Oxo-thiazolidin-2-yliden)-N-(2,6-dimethylphenyl)acetamid werden bei Raumtemperatur in 22 ml 1N Natriumhydroxid-Lösung mit 2,3 g (0,024 Mol) Dimethylsulfat tropfenweise versetzt und insgesamt 1 Stunde rühren gelassen. Danach extrahiert man das Methylierungsprodukt mit Dichlormethan und erhält nach üblicher Aufarbeitung und Kristallisation aus Methanol (Z)-(3-Methyl-4-oxo-thiazolidin-2-yliden)-N-(2,6-dimethylphenyl)acetamid vom Schmp. 250,4 °C (Zers.).

In analoger Weise, wie in Beispiel 2 beschrieben, erhält man folgende Verbindungen:

b) (Z)-(3-Methyl-4-oxo-thiazolidin-2-yliden)-N-(2-chlorphenyl)acetamid; Schmp. 193 - 194 °C (Zers.). (Methanol)

c) (Z)-(3-Methyl-4-oxo-thiazolidin-2-yliden)-N-(3-chlorphenyl)acetamid. Schmp. 275,3 °C (Zers.). (Dimethylsulfoxid = DMSO)

d) (Z)-(3-Methyl-4-oxo-thiazolidin-2-yliden)-N-(4-chlorphenyl)acetamid; Schmp. 295,5 °C (Zers.) (DMSO-Methanol)

e) (Z)-(3-Methyl-4-oxo-thiazolidin-2-yliden)-N-(2,3-dimethylphenyl)acetamid; Schmp. 205 - 207 °C (Zers.) (Ethanol)

f) (±) (Z)-(3-Methyl-4-oxo-thiazolidin-2-yliden)-N-(1-phenylethyl)acetamid; Schmp. 122 - 125 °C (Propanol-2/Petrolether).

g) (-)-(Z)-(3-Methyl-4-oxo-thiazolidin-2-yliden)-N-(1-phenylethyl)acetamid; Schmp. 55 - 60 °C

(Dichlormethan)
h) (+)-(Z)-(3-Methyl-4-oxo-thiazolidin-2-yliden)-N-(1-phenylethyl)-acetamid; Schmp. 65 - 70 °C (Dichlormethan).
i) (Z)-(3-Methyl-4-oxo-thiazolidin-2-yliden)-N-(2-chlor-6-methylphenyl)acetamid; Schmp. 244 - 245 °C (Ethanol).
j) (Z)-(3-Methyl-4-oxo-thiazolidin-2-yliden)-N-(2,6-dichlorphenyl)acetamid; Schmp. 224 - 226 °C (Toluol).
k) (Z)-(3-Methyl-4-oxo-thiazolidin-2-yliden)-N-(2,4,6 = trimethylphenyl)acetamid; Schmp. 249,8 °C (Zers.) (Ethanol).
l) (Z)-(3-Methyl-4-oxo-thiazolidin-2-yliden)-N-(4-brom-2,6-dimethylphenyl)acetamid; Schmp. 263 °C (Methanol).
m) (Z)-(3-Methyl-4-oxo-thiazolidin-2-yliden)-N-methyl-N-(2-methylphenyl)acetamid; Schmp. 114 - 115 °C (Diisopropylether).
n) (Z)-(3-Methyl-4-oxo-thiazolidin-2-yliden)-N-methyl-N-(3-methylphenyl)acetamid; Schmp. 117 - 118 °C (Diisopropylether).
o) (Z)-(3-Methyl-4-oxo-thiazolidin-2-yliden)-N-methyl-N-(2,6-dimethylphenyl)acetamid; Schmp. 153 - 154 °C (Diisopropylether).
p) (Z)-(3-Ethyl-4-oxo-thiazolidin-2-yliden)-N-(2,6-dimethylphenyl)acetamid; Schmp. 282,3 °C (Zers.) (Propranol-2).
q) (Z)-(3-Allyl-4-oxo-thiazolidin-2-yliden)-N-(2,6-dimethylphenyl)acetamid; Schmp. 250,5 °C (Zers.), (Propanol 2/Wasser).
r) (Z)-(3-Benzyl-4-oxo-thiazolidin-2-yliden)-N-(2,6-dimethylphenyl)acetamid; Schmp. 182 - 184 °C. (Toluol).
s) 3-Methyl-2-[(3-methyl-4-oxo-thiazolidin-2-yliden)acetylamino] benzoesäureethylester; Schmp. 167-168 °C (THF/Petrolether)
t) (Z)-N-(2-Methoxy-6-methyl-phenyl)-(3-methyl-4-oxo-thiazolidin-2-yliden)acetamid; Schmp. 158-160 °C (Isopropanol)
u) (Z)-N-(2-Methylthio-phenyl)-(3-methyl-4-oxo-thiazolidin-2-yliden) acetamid; Schmp. 177-178 °C (Toluol)
v) Z-(3-Methyl-4-oxo-thiazolidin-2-yliden)-N-(3,4,5-trimethoxyphenyl) acetamid; Schmp. 224-225 °C (Isopropanol)
w) (Z)-N-(2-Methyl-6-nitro-phenyl)-(3-methyl-4-oxo-thiazolidin-2-yliden)acetamid; Schmp. 218-219 °C (Tolual/Aceton)
x) (Z)-N-(2-Diethylaminoethyl)-N-(2,6-xylyl)-(3-methyl-4-oxo-thiazolidin-2-yliden)acetamid • HBr; Schmp. 261,5 °C (Ethanol)
y) Z-(3-Methyl-4-oxo-thiazolidin-2-yliden)acetanilid; Schmp. 212-215 °C (Ethanol)
z) Z-N-(4-Chlor-2-nitrophenyl)-(3-methyl-4-oxo-thiazolidin-2-yliden) acetamid; Schmp. 264-265 °C (DMF/$H_2O$)
aa) 2-Hydroxy-4-[(3-methyl-4-oxo-thiazolidin-2-yliden)acetylamino] benzoesäureethylester; Schmp. 236-237 °C (DMF/$H_2O$)
bb) (Z)-N-(3-Diethylaminopropyl)-N-(2,6-xylyl)-(3-methyl-4-oxothiazolidin-2-yliden )-acetamid • HCl; Schmp. 222-223 °C (Isopropanol)
cc) (Z)-N-(2-Dimethylaminoethyl)-N-(2,6-xylyl)-(3-methyl-4-oxothiazolidin-2-yliden)acetamid • HCl; Schmp. 268,8 °C (Ethanol)
dd) (Z)-N-(3-Dimethylaminopropyl)-N-(2,6-xylyl)-(3-methyl-4-oxothiazolidin-2-yliden)-acetamid • Hydrobromid; Schmp. 253,6 °C (Ethanol)
ee) (Z)-3-Methyl-2-[(3-methyl-4-oxo-thiazolidin-2-yliden)acetylamino]-benzoesäure; Schmp. 186,9 °C (Dichlormethan)
ff) (Z)-(4-Oxo-3-phenyl-thiazolidin-2-yliden)-N-(2,6-xylyl) acetamid; Schmp. 228,3 °C (Ethanol)
gg) (Z)-N-(2-Chlor-6-methylphenyl)-(4-oxo-3-phenyl-thiazolidin-2-yliden)acetamid; Schmp. 213-214 °C (Ethanol)
hh) Z-(3,5-Dimethyl-4-oxo-thiazolidin-2-yliden)-N-(2,6-xylyl)-acetamid; Schmp. 220-222 °C (Ethanol)

## Pharmakologische Vergleichsversuche

### Methodik

### Allgemeines

Bei allen Substanzen wurde in den Krampftests als Vehikel 0,8 % Methocelschleim benutzt. Die Prämedikationszeit betrug 30 Minuten, sofern nicht anders angegeben, und die Applikation erfolgte i.g. in 20 ml/kg Körpergewicht.

Versuchstiere waren männliche Mäuse (NMRI) mit einem Körpergewicht von 18 bis 24 g, die unter konditionierten Bedingungen gehalten wurden. 24 Stunden (falls nicht anders angegeben) vor Versuchsbeginn wurde das Futter entzogen, das Trinkwasser jedoch bis zu Beginn des Versuchs belassen.

Bei den audiogenen Krämpfen wurden DBA/2J-Mäuse männlichen Geschlechts im Gewicht von 5 - 10 g verwendet, die am Tag vor dem Versuch bezogen und nicht nüchtern gesetzt wären.

Pro Dosierung wurden jeweils 10 Tiere dem Test unterzogen.

Als Positivstandard wurden Phenytoin, Valproat, GABA und Baclofen verwendet.

Testmodelle

1. Isoniazidkrampf

Als Konvulsivum wurde Isonicotinsäure-hydrazid (Isoniazid) in einer Dosierung von 250 mg/kg s.c. benutzt, wobei die Beobachtungszeit 90 min betrug.

2. Strychninkrampf

Als Konvulsivum wurde Strychnin nitric. cryst. in einer Dosis von 1,1 mg/kg s.c. angewendet, wobei die Beobachtungszeit 20 min p.a. betrug.

3. Elektroschock

Für den Elektroschock wurde das HSE-Schock-Reizgerät Typ 207 verwendet.
Zur Übertragung des Stromstoßes wurden Elektroden in Form von Ohrclips an den Ohren der Maus befestigt. Die Dauer des Schocks betrug 0,3 s, die Stromstärke 35 mA (Spannung selbstregelnd).
Als Positivstandard wurde Phenytoin-Natrium (EPANUTIN$^{(R)}$) in einer Dosis von 50 mg/kg i.g. bzw. s.c. angewendet. Die Beobachtungszeit war auf 5 min festgelegt.

4. Pentetrazolkrampf

Als Konvulsivum wurden 140 mg/kg Pentetrazol s.c. appliziert. Die Beobachtungszeit betrug 20 min.

5. Pikrotoxinkrampf

Pikrotoxin wurde in einer Dosis von 15 mg/kg s.c. verabreicht. Beobachtet wurden die Tiere 20 min.

6. Bicucullinkrampf

Krampfmittel war Bicucullin purum. Die Dosis betrug 3,0 mg/kg s.c.. Die Beobachtungszeit betrug 20 min.

7. Semicarbazidkrampf

Semicarbazid-Hydrochlorid wurde als Krampfmittel in einer Dosis von 1000 mg/kg s.c. verwendet. Die Beobachtungszeit betrug jeweils 90 min p.a.

8. 3-Mercaptopropionsäure

3-Mercaptopropionsäure wurde mit physiologischer NaCl-Lösung verdünnt, mit 6 mol/l NaOH auf pH 7 eingestellt und mit NaCl-Lösung auf das Endvolumen gebracht (10 ml/kg). Die Beobachtungszeit betrug 30 min.

9. Audiogene Krämpfe

Die Tiere wurden mit einem 10,9 KHz-Ton von 110 dBA 30 Sekunden lang beschallt. Die Beobach-

tungszeit betrug 1 min.

TABELLE I

VERGLEICHENDE UNTERSUCHUNGEN DER ANTIKONVULSIVEN WIRKUNG IN DEN KRAMPFMODELLEN 1. - 9.

| Substanz Beispiel Nr. | $ED_{50}$ (mg/kg i.g., Maus ♂) Elektroschock | Pentetrazolkrampf | Semicarbacidkrampf | Pikrotoxinkrampf | Strychninkrampf | 3-MPA-krampf | Isoniazidkrampf | Bicucullinkrampf | audiog. Krämpfe |
|---|---|---|---|---|---|---|---|---|---|
| 2 a) | 13,6 | 19,85 | 14,06 | 11,30 | 14,88 | 8,7 | 5,32 | 2,68 | 2,9 |
| 2 i) | 6,47 | 1,90 | 1,59 | 2,34 | 5,09 | 1,29 | 1,24 | 0,9 | 3,0 |
| 2 j) | 4,03 | 1,80 | 3,41 | 1,52 | 5,68 | 1,73 | 5,0 | 0,8 | 2,0 |
| Vergleichs=substanzen | | | | | | | | | |
| Phenytoin | 50 (ED 100) 12,5 (i.v.) | 50 (ED 100) | 50 (ED 90) | > 200 | inaktiv | -- | 38 | 12,5 | 2,3 |
| Valproate | 200 (ED 40) | ca. 250 | 100 | inaktiv | inaktiv | 154 | 135 | -- | -- |
| GABA | inaktiv | 500 | 60 | inaktiv | inaktiv | -- | -- | -- | -- |
| Baclofen[1)] | 20 | 100 | 4 | 30 | 8,5 | 20 | 2,0 | 20 | 5 |

[1)] Nebenwirkungen in allen Modellen

Akute Toxizität und therapeutischer Index

Die Bestimmung der akuten Toxizität wurde an männlichen Mäusen (NMRI) mit einem Körpergewicht von 18 - 23 g durchgeführt. Alle Versuchstiere wurden 20 Stunden vor Versuchsbeginn nüchtern gesetzt. Wasser stand ad libitum zur Verfügung. Zu jeder Dosierungsgruppe gehörten 4 Tiere. Die Dosenfolge war logarithmisch. Die Prüfsubstanzen wurden als Suspension in 0,88 %igem Methocel intragastral verabreicht. Das Applikationsvolumen betrug 20 ml/kg Körpergewicht. Die Tiere wurden insgesamt 7 Tage beobachtet.

Tabelle II

| Substanz Beispiel Nr. | LD 50 mg/kg i.g. (Maus ♂) | Therapeutischer Index LD 50 / ED 50 *) |
|---|---|---|
| 2 a) | 1980 | 99,8 - 738,8 |
| 2 i) | > 1600 | > 243,3 - 1777,8 |
| 2 j) | 1600 | 281,6 - 2000 |
| Valproat | 2000 | 8 - 14,8 |
| GABA | > 5000 | > 10 - 80 |
| Phenytoin | 490 | 2,5 - 213 |
| Baclofen | 200 | 2 - 100 |

*) jeweils für den größten und kleinsten $ED_{50}$-Wert errechnet

Aus den Tabellen I und II geht das überlegene Wirkungsspektrum der Verbindungen einerseits (Tabelle I) und die außerordentlich günstige relative Sicherheit aufgrund der therapeutischen Quotienten (Tabelle II) andererseits hervor.

**Patentansprüche**

**1.** 4-Oxo-thiazolidin-2-yliden-acetamidderivate der allgemeinen Formel I

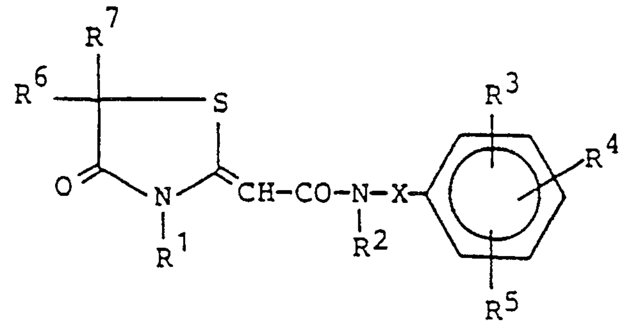

(I),

in welcher
$R^1$ ein Wasserstoffatom, einen gesättigten oder ungesättigten Alkylrest mit 1 bis 3 Kohlenstoffatomen, einen Phenyl- oder Benzylrest, $R^2$ ein Wasserstoffatom, eine Methyl- oder Ethylgruppe oder einen Dialkylaminoalkylrest mit insgesamt bis zu 7 Kohlenstoffatomen, X einen Bindestrich oder eine geradkettige oder verzweigte Alkylen kette mit 1 - 3 Kohlenstoffatomen und $R^3$, $R^4$ und $R^5$, die gleich oder verschieden sein können, ein Wasserstoffatom, eine Methyl-, Ethyl-, Methoxy-, Ethoxy-, Methylthio- oder Ethylthiogruppe, wobei zwei benachbarte Reste gemeinsam auch eine Methylendioxygruppe bilden können, ein Fluor-, Chlor-, Brom-, Jodatom, eine Hydroxygrupne oder eine Nitrogruppe oder eine Carboxylgruppe oder einen Niederalkoxycarbonylrest mit bis zu 5 Kohlenstoffatomen und $R^6$ und $R^7$ Wasserstoff, eine Methyl- oder eine Ethylgruppe bedeutet.

**2.** 4-Oxo-thiazolidin-2-yliden-acetamid-derivate nach Anspruch 1, bei welchen in der allgemeinen Formel I $R^1$ ein Wasserstoffatom, einen Methyl-, Ethyl-, Allyl- oder Benzylrest, $R^2$ ein Wasserstoffatom oder einen Methylrest, X einen Bindestrich oder eine Ethylidengruppe und $R^3$, $R^4$ und $R^5$, die gleich oder verschieden sein können, ein Wasserstoffatom, einen Methylrest oder ein Chlor- oder Bromatom und $R^6$ und $R^7$ ein Wasserstoffatom bedeutet.

**3.** (Z)-(3-Methyl-4-oxo-thiazolidin-2-yliden)-N-(2,6-dimethylphenyl)acetamid.

**4.** (Z)-(3-Methyl-4-oxo-thiazolidin-2-yliden)-N-(2-chlor-6-methyl-phenyl) acetamid.

**5.** (Z)-(3-Methyl-4-oxo-thiazolidin-2-yliden)-N-(2,6-dichlorphenyl)acetamid.

**6.** Verfahren zur Herstellung von 4-Oxo-thiazolidin-2-yliden-acetamid-derivaten nach Anspruch 1 bis 5 dadurch gekennzeichnet, daß man entweder
a) eine Verbindung der allgemeinen Formel II

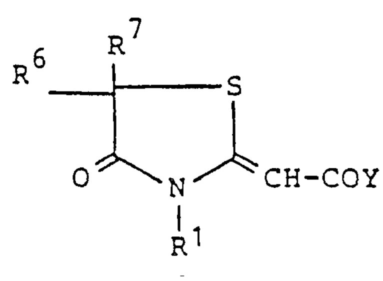

(II),

in welcher $R^1$, $R^6$ und $R^7$ die obengenannte Bedeutung haben und y eine reaktive Gruppe darstellt in an sich bekannter Weise mit einer Verbindung der allgemeinen Formel (III)

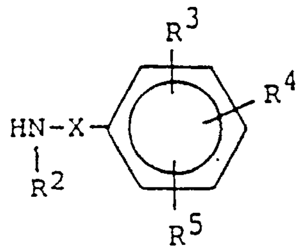 (III),

in welcher die Reste $R^2$, $R^3$, $R^4$ und $R^5$ sowie X die obengenannte Bedeutung haben, umsetzt und gegebenenfalls anschließend N-alkyliert oder
b) eine Verbindung der allgemeinen Formel IV

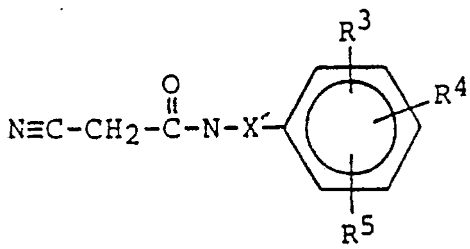 (IV),

in welcher die Reste $R^3$, $R^4$ und $R^5$ und X die obengenannte Bedeutung haben, mit einem Thioglykolsäureester der allgemeinen Formel V

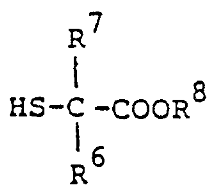 (V),

in welcher $R^6$ und $R^7$ die obengenannte Bedeutung haben und $R^8$ einen niederen Alkylrest mit bis zu 5 Kohlenstoffatomen darstellt,
umsetzt, die erhaltenen Verbindungen der allgemeinen Formel VI

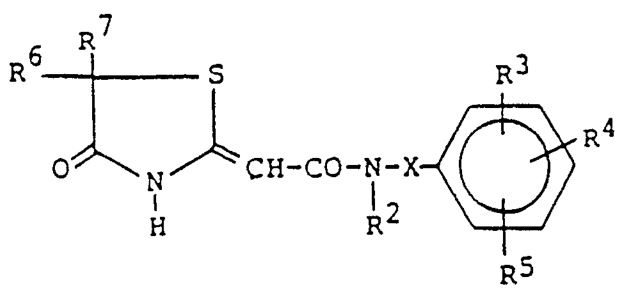 (VI),

in welcher $R^2$, X, $R^3$, $R^4$, $R^5$, $R^6$ und $R^7$ die obengenannte Bedeutung haben, gewünschtenfalls in an sich bekannter Weise anschließend N-alkyliert und die so erhaltenen Verbindungen der allgemeinen Formel I in an sich bekannter Weise isoliert.

7. Arzneimittel, dadurch gekennzeichnet daß sie als Wirkstoff mindestens eine Verbindung gemäß Anspruch 1 bis 5 sowie übliche Trägerstoffe enthalten.

**Patentansprüche für folgenden Vertragsstaat: AT**

1. Verfahren zur Herstellung von 4-Oxo-thiazolidin-2-yliden-acetamidderivaten der allgemeinen Formel I

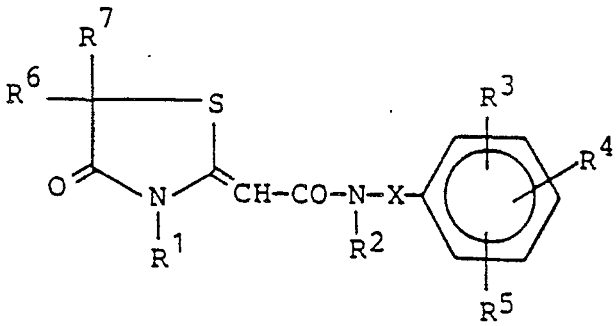

(I),

in welcher
$R^1$ ein Wasserstoffatom, einen gesättigten oder ungesättigten Alkylrest mit 1 bis 3 Kohlenstoffatomen, einen Phenyl- oder Benzylrest, $R^2$ ein Wasserstoffatom, eine Methyl- oder Ethylgruppe oder einen Dialkylaminoalkylrest mit insgesamt bis zu 7 Kohlenstoffatomen, X einen Bindestrich oder eine geradkettige oder verzweigte Alkylenkette mit 1 - 3 Kohlenstoffatomen und $R^3$, $R^4$ und $R^5$, die gleich oder verschieden sein können, ein Wasserstoffatom, eine Methyl-, Ethyl-, Methoxy-, Ethoxy-, Methylthio- oder Ethylthiogruppe, wobei zwei benachbarte Reste gemeinsam auch eine Methylendioxygruppe bilden können, ein Fluor-, Chlor-, Brom-, Jodatom, eine Hydroxygruppe oder eine Nitrogruppe oder eine Carboxylgruppe oder einen Niederalkoxycarbonylrest mit bis zu 5 Kohlenstoffatomen und $R^6$ und $R^7$ Wasserstoff, eine Methyl- oder eine Ethylgruppe bedeutet,
dadurch gekennzeichnet, daß man entweder

a) eine Verbindung der allgemeinen Formel II

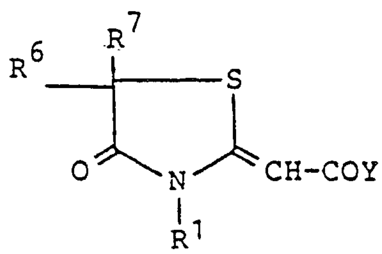

(II),

in welcher $R^1$, $R^6$ und $R^7$ die obengenannte Bedeutung haben und y eine reaktive Gruppe darstellt in an sich bekannter Weise mit einer Verbindung der allgemeinen Formel (III)

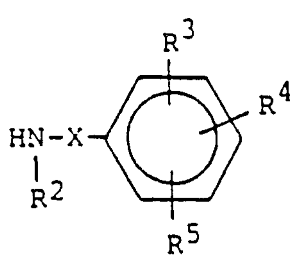

(III),

in welcher die Reste $R^2$, $R^3$, $R^4$ und $R^5$ sowie X die obengenannte Bedeutung haben, umsetzt und gegebenenfalls anschließend N-alkyliert oder

b) eine Verbindung der allgemeinen Formel IV

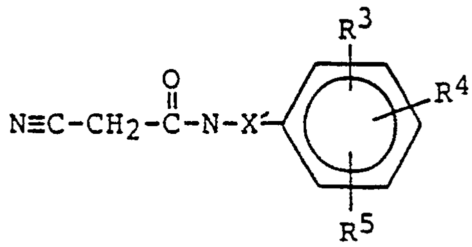

(IV),

in welcher die Reste $R^3$, $R^4$ und $R^5$ und X die obengenannte Bedeutung haben, mit einem Thioglykolsäureester der allgemeinen Formel V

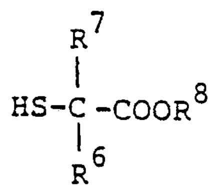

(V),

in welcher $R^6$ und $R^7$ die obengenannte Bedeutung haben und $R^8$ einen niederen Alkylrest mit bis zu 5 Kohlenstoffatomen darstellt,
umsetzt, die erhaltenen Verbindungen der allgemeinen Formel VI

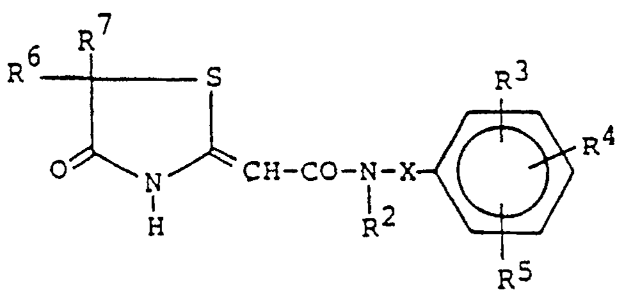

(VI),

in welcher $R^2$, X, $R^3$, $R^4$, $R^5$, $R^6$ und $R^7$ die obengenannte Bedeutung haben,
gewünschtenfalls in an sich bekannter Weise anschließend N-alkyliert und die so erhaltenen Verbindungen der allgemeinen Formel I in an sich bekannter Weise isoliert.

2. Verfahren gemäß Anspruch 1 zur Herstellung von Verbindungen gemäß der allgemeinen Formel I bei welchen in der allgemeinen Formel I $R^1$ ein Wasserstoffatom, einen Methyl-, Ethyl-, Allyl- oder Benzylrest, $R^2$ ein Wasserstoffatom oder einen Methylrest, X einen Bindestrich oder eine Ethylidengruppe und $R^3$, $R^4$ und $R^5$, die gleich oder verschieden sein können, ein Wasserstoffatom, einen Methylrest oder ein Chlor- oder Bromatom und $R^6$ und $R^7$ ein Wasserstoffatom bedeutet.

3. Verfahren gemäß Anspruch 1 zur Herstellung von (Z)-(3-Methyl-4-oxo-thiazolidin-2-yliden)-N-(2,6-dimethylphenyl) acetamid.

4. Verfahren gemäß Anspruch 1 zur Herstellung von (Z)-(3-Methyl-4-oxo-thiazolidin-2-yliden)-N-(2-chlor-6-methyl-phenyl) acetamid.

5. Verfahren gemäß Anspruch 1 zur Herstellung von (Z)-(3-Methyl-4-oxo-thiazolidin-2-yliden)-N-(2,6-dichlorphenyl) acetamid.

6. Verwendung einer nach Anspruch 1-5 hergestellten Verbindung zur Herstellung eines Arzneimittels zur antiepileptischen Therapie.

## Claims

1. 4-oxo-thiazolidine-2-ylidene-acetamide derivates of the general formula I

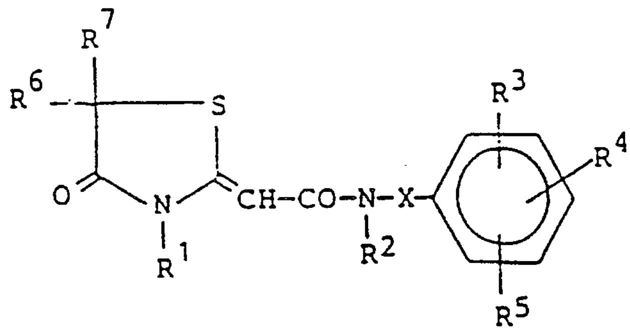

(I),

in which
$R^1$ signifies a hydrogen atom, a saturated or unsaturated alkyl residue with 1 to 3 carbon atoms, a phenyl residue or benzyl residue, $R_2$ a hydrogen atom, a methyl group or ethyl group or a dialkyl aminoalkyl residue with, in all, up to 7 carbon atoms, X a hyphen or a straight-chain or branched alkylene chain with 1 - 3 carbon atoms and $R^3$, $R^4$ and $R^5$, which can be the same or different, signify a hydrogen atom, a methyl group, ethyl group, methoxy group, ethoxy group, methylthio group or ethylthio group, in which case two adjacent residues together can also form a methylene dioxy group, a fluorine atom, chlorine atom, bromine atom, iodine atom, a hydroxy group or a nitro group or a carboxyl group or a lower alkoxy carbonyl residue with up to 5 carbon atoms and $R^6$ and $R^7$ signify hydrogen, a methyl group or an ethyl group.

**2.** 4-oxo-thiazolidine-2-ylidene-acetamide-derivates according to claim 1, in which in the general formula I $R^1$ signifies a hydrogen atom, a methyl residue, ethyl residue, allyl residue or benzyl residue, $R^2$ a hydrogen atom or a methyl residue, X a hyphen or an ethylidene group, and $R^3$, $R^4$ and $R^5$, which can be same or different, signify a hydrogen atom, a methyl residue or a chlorine atom or bromine atom, and $R^6$ and $R^7$ signify a hydrogen atom.

**3.** (Z)-(3-methyl-4-oxo-thiazolidine-2-ylidene)-N-(2,6-dimethylphenyl)acetamide.

**4.** (Z)-(3-methyl-4-oxo-thiazolidine-2-ylidene)-N-(2-chloro-6-methyl-phenyl) acetamide.

**5.** (Z)-(3-methyl-4-oxo-thiazolidine-2-ylidene)-N-(2,6-dichlorophenyl)acetamide.

**6.** Method for the preparation of 4-oxo-thiazolidine-2-ylidene-acetamide-derivates according to claim 1 to 5, characterised in that either
a) a compound of the general formula II

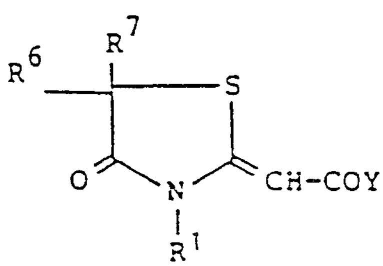

(II),

in which $R^1$, $R^6$ and $R^7$ have the above-mentioned signification and y represents a reactive group, is caused to react in a manner known per se with a compound of the general formula (III)

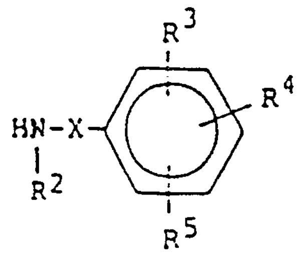

(III),

in which the residues $R^2$, $R^3$, $R^4$ and $R^5$ and also X have the above-mentioned signification, and, if applicable, subsequently is N-alkylated, or
b) a compound of the general formula IV

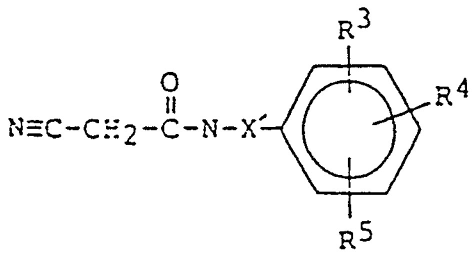

(IV),

in which the residues $R^3$, $R^4$ and $R^5$ and X have the above-mentioned signification, is caused to react with a thioglycolic acid ester of the general formula V

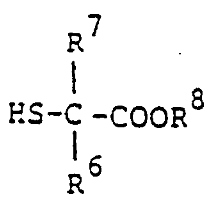

(V),

in which $R^6$ and $R^7$ have the above-mentioned signification and $R^8$ represents a lower alkyl residue with up to 5 carbon atoms, the compounds obtained of the general formula VI

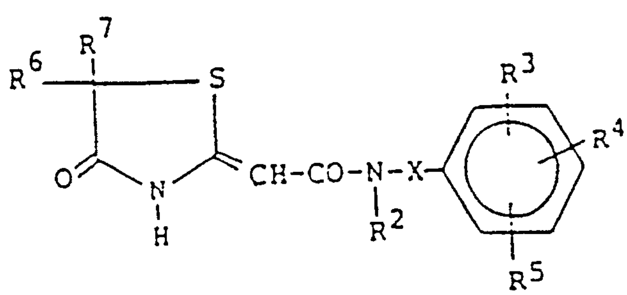

(VI),

in which $R^2$, X, $R^3$, $R^4$, $R^5$, $R^6$ and $R^7$ have the above-mentioned signification, if desired, are subsequently N-alkylated in a manner known per se and the compounds thus obtained of the general formula I are isolated in a manner known per se.

7. Pharmaceuticals, characterised in that they contain as an active ingredient at least one compound according to claim 1 to 5 as well as conventional carrier substances.

**Claims for the Following Contracting State: AT**

**1.** Method for the preparation of 4-oxo-thiazolidine-2-ylidene-acetamide derivates of the general formula I

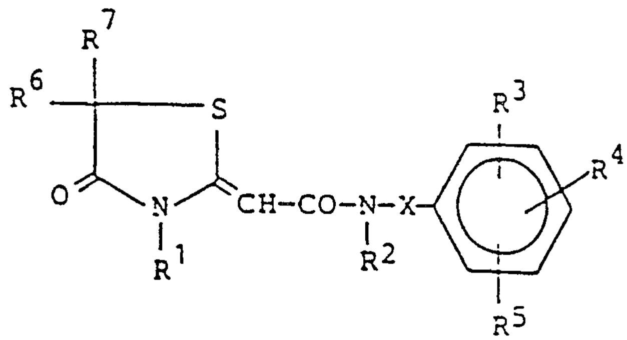

(I),

in which
$R^1$ signifies a hydrogen atom, a saturated or unsaturated alkyl residue with 1 to 3 carbon atoms, a phenyl residue or benzyl residue, $R_2$ a hydrogen atom, a methyl group or ethyl group or a dialkyl aminoalkyl residue with, in all, up to 7 carbon atoms, X a hyphen or a straight-chain or branched alkylene chain with 1 - 3 carbon atoms and $R^3$, $R^4$ and $R^5$, which can be the same or different, signify a hydrogen atom, a methyl group, ethyl group, methoxy group, ethoxy group, methylthio group or ethylthio group, in which case two adjacent residues together can also form a methylene dioxy group, a fluorine atom, chlorine atom, bromine atom, iodine atom, a hydroxy group or a nitro group or a carboxyl group or a lower alkoxy carbonyl residue with up to 5 carbon atoms and $R^6$ and $R^7$ signify hydrogen, a methyl group or an ethyl group, characterised in that either

a) a compound of the general formula II

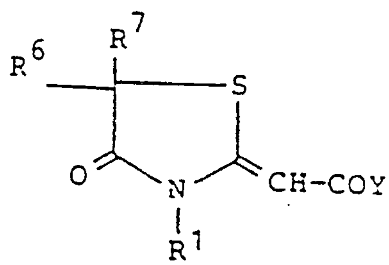

(II),

in which $R^1$, $R^6$ and $R^7$ have the above-mentioned signification and y represents a reactive group, is caused to react in a manner known per se with a compound of the general formula (III)

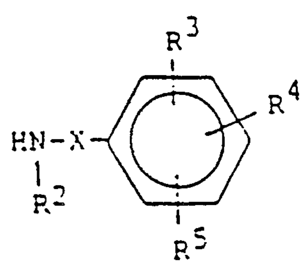

(III),

in which the residues $R^2$, $R^3$, $R^4$ and $R^5$ and also X have the above-mentioned signification, and, if applicable, subsequently is N-alkylated, or

b) a compound of the general formula IV

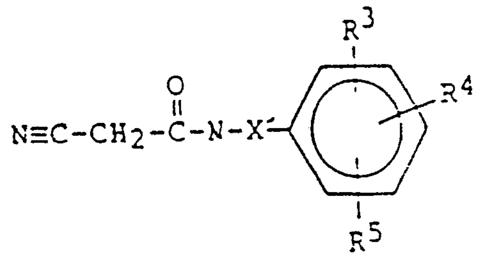

(IV),

in which the residues $R^3$, $R^4$ and $R^5$ and X have the above-mentioned signification, is caused to react with a thioglycolic acid ester of the general formula V

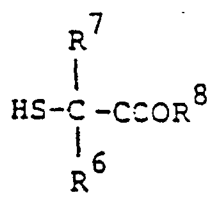

(V),

in which $R^6$ and $R^7$ have the above-mentioned signification and $R^8$ represents a lower alkyl residue with up to 5 carbon atoms, the compounds obtained of the general formula VI

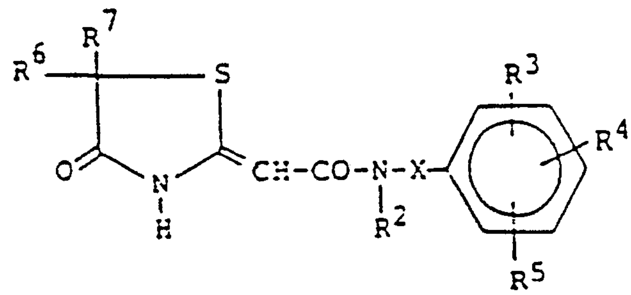

(VI),

in which $R^2$, X, $R^3$, $R^4$, $R^5$, $R^6$ and $R^7$ have the above-mentioned signification, if desired, are subsequently N-alkylated in a manner known per se and the compounds thus obtained of the general formula I are isolated in a manner known per se.

**2.** Method according to claim 1 for the preparation of compounds according to the general formula I, in which in the general formula I $R^1$ signifies a hydrogen atom, a methyl residue, ethyl residue, allyl residue or benzyl residue, $R^2$ a hydrogen atom or a methyl residue, X a hyphen or an ethylidene group and $R^3$, $R^4$ and $R^5$, which can be same or different, signify a hydrogen atom, a methyl residue or a chlorine atom or bromine atom and $R^6$ and $R^7$ signify a hydrogen atom.

**3.** Method according to claim 1 for the preparation of (Z)-(3-methyl-4-oxo-thiazolidine-2-ylidene)-N-(2,6-dimethylphenyl)acetamide.

**4.** Method according to claim 1 for the preparation of (Z)-(3-methyl-4-oxo-thiazolidine-2-ylidene)-N-(2-chloro-6-methyl-phenyl) acetamide.

**5.** Method according to claim 1 for the preparation of (Z)-(3-methyl-4-oxo-thiazolidine-2-ylidene)-N-(2,6-dichlorophenyl)acetamide.

**6.** Use of a compound prepared according to claim 1 - 5 for the manufacture of a pharmaceutical for antiepileptic therapy.

**Revendications**

1. Dérivés de 4-oxo-thiazolidine-2-ylidène-acétamide répondant à la formule générale I:

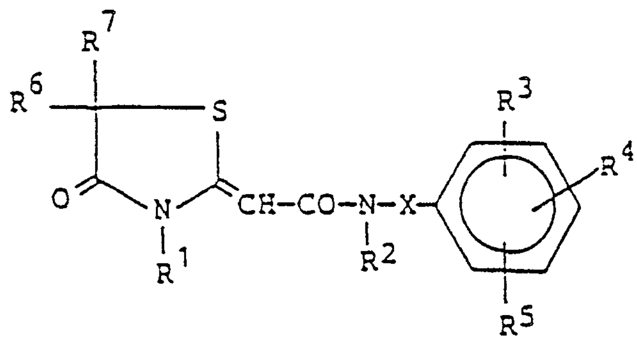

(I)

dans laquelle:

| | |
|---|---|
| $R^1$ | représente un atome d'hydrogène, un radical alkyle saturé ou insaturé renfermant de 1 à 3 atomes de carbone, un radical phényle ou benzyle; |
| $R^2$ | représente un atome d'hydrogène, un groupe méthyle ou éthyle ou un radical dialkylaminoalkyle renfermant au total jusqu'à 7 atomes de carbone; |
| X | représente un trait de liaison ou une chaîne alkylène droite ou ramifiée renfermant de 1 à 3 atomes de carbone; et |
| $R^3$, $R^4$ et $R^5$, | qui peuvent être identiques ou différents, représentent un atome d'hydrogène, un groupe méthyle, éthyle, méthoxy, éthoxy, méthylthio ou éthylthio, deux radicaux voisins pouvant également former ensemble un groupe méthylènedioxy, un atome de fluor, de chlore, de brome, d'iode, un groupe hydroxy ou un groupe nitro ou un groupe carboxyle ou un radical alkoxycarbonyle inférieur renfermant jusqu'à 5 atomes de carbone; et |
| $R^6$ et $R^7$ | représentent un atome d'hydrogène, un groupe méthyle ou un groupe éthyle. |

2. Dérivés de 4-oxo-thiazolidine-2-ylidène-acétamide selon la revendication 1, caractérisés en ce que, dans la formule générale I: $R^1$ représente un atome d'hydrogène, un radical méthyle, éthyle, allyle ou benzyle; $R^2$ représente un atome d'hydrogène ou un radical méthyle; X représente un trait de liaison ou un groupe éthylidène; et $R^3$, $R^4$ et $R^5$ qui peuvent être identiques ou différents, représentent un atome d'hydrogène, un radical méthyle ou un atome de chlore ou de brome; et $R^6$ et $R^7$ représentent un atome d'hydrogène.

3. (Z)-(3-méthyl-4-oxo-thiazolidine-2-ylidène)-N-(2,6-diméthylphényl)acétamide.

4. (Z)-(3-méthyl-4-oxo-thiazolidine-2-ylidène)-N-(2-chloro-6-méthylphényl)acétamide.

5. (Z)-(3-méthyl-4-oxo-thiazolidine-2-ylidène)-N-(2,6-dichlorophényl)acétamide.

6. Procédé de préparation de dérivés de 4-oxo-thiazolidine-2-ylidène-acétamide selon les revendications 1 à 5, caractérisé en ce que soit:

a) on fait réagir un dérivé de formule générale II:

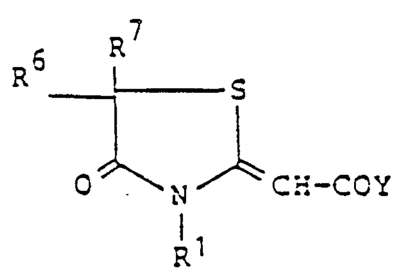

(II)

dans laquelle:

$R^1$, $R^6$ et $R^7$ ont la signification indiquée ci-dessus; et

Y représente un groupe réactif;

d'une façon connue en soi avec un dérivé de formule générale (III):

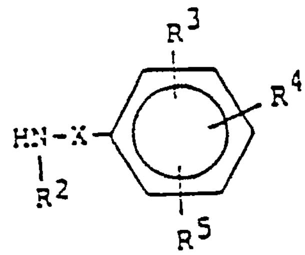

(III)

dans laquelle les radicaux $R^2$, $R^3$, $R^4$ et $R^5$ ainsi que X ont la signification indiquée ci-dessus, et, le cas échéant, on procède à une alkylation sur l'atome de N;

soit:

b) on fait réagir un dérivé de formule générale IV:

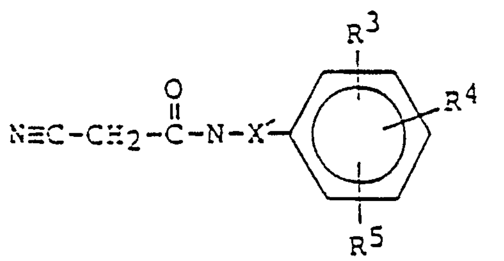

(IV)

dans laquelle les radicaux $R^3$, $R^4$ et $R^5$ et X ont la signification indiquée ci-dessus;

avec un ester d'acide thioglycolique répondant à la formule générale V:

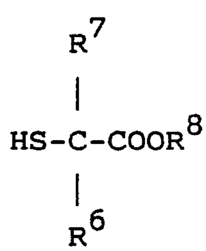

(V)

dans laquelle:

$R^6$ et $R^7$ ont la signification indiquée ci-dessus; et

$R^8$ représente un radical alkyle inférieur renfermant jusqu'à 5 atomes de carbone,

le cas échéant, on procède ensuite d'une façon connue en soi à une alkylation sur l'atome de N des dérivés obtenus répondant à la formule générale VI:

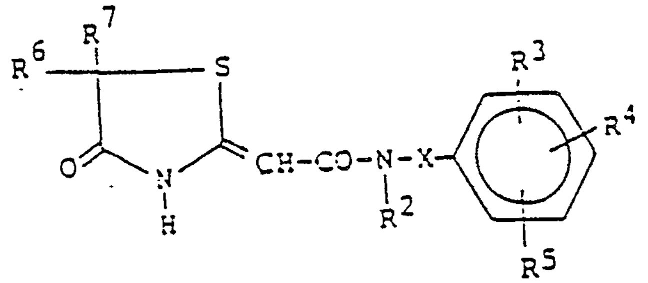

(VI)

dans laquelle $R^2$, X, $R^3$, $R^4$, $R^5$, $R^6$ et $R^7$ ont la signification indiquée; et
d'une façon connue en soi, on sépare les dérivés ainsi obtenus répondant à la formule générale I.

7. Médicament caractérisé en ce qu'il contient comme substance active au moins un dérivé selon les revendications 1 à 5, ainsi que les supports usuels.

**Revendications pour l'Etat contractant suivant: AT**

1. Procédé de préparation de dérivés de 4-oxo-thiazolidine-2-ylidène-acétamide répondant à la formule générale I:

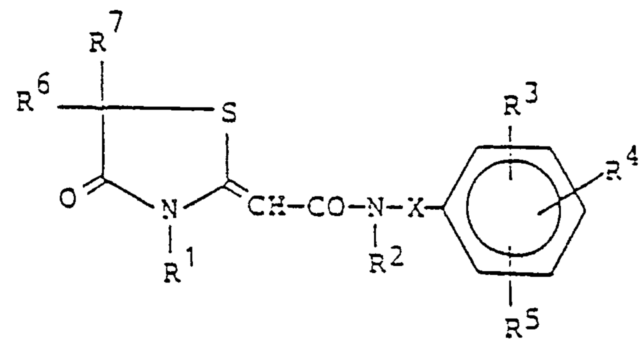

(I)

dans laquelle:

| | |
|---|---|
| $R^1$ | représente un atome d'hydrogène, un radical alkyle saturé ou insaturé renfermant de 1 à 3 atomes de carbone, un radical phényle ou benzyle; |
| $R^2$ | représente un atome d'hydrogène, un groupe méthyle ou éthyle ou un radical dialkylaminoalkyle renfermant au total jusqu'à 7 atomes de carbone; |
| X | représente un trait de liaison ou une chaîne alkylène droite ou ramifiée renfermant de 1 à 3 atomes de carbone; et |
| $R^3$, $R^4$ et $R^5$, | qui peuvent être identiques ou différents, représentent un atome d'hydrogène, un groupe méthyle, éthyle, méthoxy, éthoxy, méthylthio ou éthylthio, deux radicaux voisins pouvant également former ensemble un groupe méthylènedioxy, un atome de fluor, de chlore, de brome, d'iode, un groupe hydroxy ou un groupe nitro ou un groupe carboxyle ou un radical alkoxycarbonyle inférieur renfermant jusqu'à 5 atomes de carbone; et |
| $R^6$ et $R^7$ | représentent un atome d'hydrogène, un groupe méthyle ou un groupe éthyle, |

caractérisé en ce que soit:

a) on fait réagir un dérivé de formule générale II:

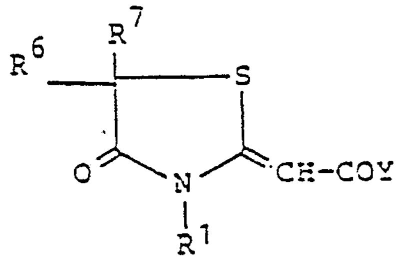

(II)

dans laquelle:

$R^1$, $R^6$ et $R^7$ ont la signification indiquée ci-dessus; et

Y représente un groupe réactif;

d'une façon connue en soi avec un dérivé de formule générale (III):

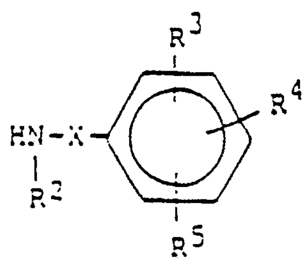

(III)

dans laquelle les radicaux $R^2$, $R^3$, $R^4$ et $R^5$ ainsi que X ont la signification indiquée ci-dessus, et, le cas échéant, on procède à une alkylation sur l'atome de N;

soit:

b) on fait réagir un dérivé de formule générale IV:

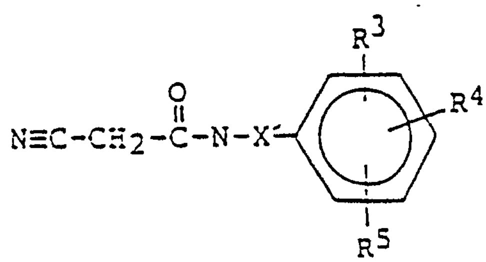

(IV)

dans laquelle les radicaux $R^3$, $R^4$ et $R^5$ et X ont la signification indiquée ci-dessus;

avec un ester d'acide thioglycolique répondant à la formule générale V:

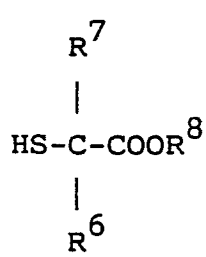

(V)

dans laquelle:

$R^6$ et $R^7$ ont la signification indiquée ci-dessus; et

$R^8$ représente un radical alkyle inférieur renfermant jusqu'à 5 atomes de carbone,

le cas échéant, on procède ensuite d'une façon connue en soi à une alkylation sur l'atome de N des dérivés obtenus répondant à la formule générale VI:

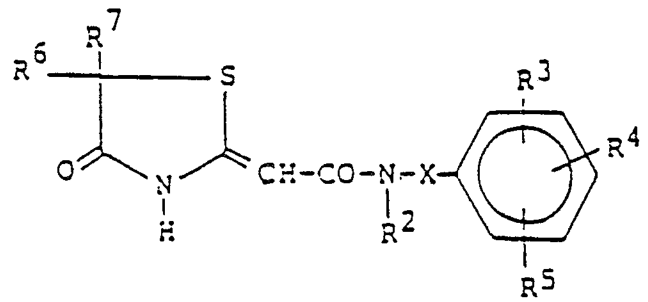

(VI)

dans laquelle $R^2$, X, $R^3$, $R^4$, $R^5$, $R^6$ et $R^7$ ont la signification indiquée; et
d'une façon connue en soi, on sépare les dérivés ainsi obtenus répondant à la formule générale I.

2. Procédé selon la revendication 1 de préparation de dérivés de 4-oxo-thiazolidine-2-ylidène-acétamide répondant à la formule générale I, dans lesquels, dans la formule générale I: $R^1$ représente un atome d'hydrogène, un radical méthyle, éthyle, allyle ou benzyle; $R^2$ représente un atome d'hydrogène ou un radical méthyle; X représente un trait de liaison ou un groupe éthylidène; et $R^3$, $R^4$ et $R^5$ qui peuvent être identiques ou différents, représentent un atome d'hydrogène, un radical méthyle ou un atome de chlore ou de brome; et $R^6$ et $R^7$ représentent un atome d'hydrogène.

3. Procédé selon la revendication 1 de préparation de (Z)-(3-méthyl-4-oxo-thiazolidine-2-ylidène)-N-(2,6-diméthylphényl)acétamide.

4. Procédé selon la revendication 1 de préparation de (Z)-(3-méthyl-4-oxo-thiazolidine-2-ylidène)-N-(2-chloro-6-méthylphényl)acétamide.

5. Procédé selon la revendication 1 de préparation de (Z)-(3-méthyl-4-oxo-thiazolidine-2-ylidène)-N-(2,6-dichlorophényl)acétamide.

6. Utilisation d'un dérivé préparé selon les revendications 1 à 5, pour la préparation d'un médicament destiné à une thérapeutique anti-épileptique.